Europäisches Patentamt

European Patent Office

Office européen des brevets

(11) Numéro de publication: **0 311 721 B1**

## (12) FASCICULE DE BREVET EUROPEEN

(45) Date de publication de fascicule du brevet: **01.07.92**  (51) Int. Cl.⁵: **A61M 35/00**, B65D 35/36

(21) Numéro de dépôt: **87402269.2**

(22) Date de dépôt: **12.10.87**

(54) **Composition plastique pour la protection de surfaces corporelles et dispositif pour son application.**

(43) Date de publication de la demande:
**19.04.89 Bulletin 89/16**

(45) Mention de la délivrance du brevet:
**01.07.92 Bulletin 92/27**

(84) Etats contractants désignés:
**AT BE CH DE ES GB GR IT LI LU NL SE**

(56) Documents cités:
**FR-A- 1 113 147**
**FR-A- 2 467 786**
**LU-A- 67 890**
**US-A- 1 860 790**

**THE MERCK INDEX - An Encyclopedia of
Chemicals, Drugs, and Biologicals, 1983,
10ème édition, page 2452, Merck & Co., Inc.,
Rahway, N.J., US**

(73) Titulaire: **Perovitch, Philippe**
**251, avenue de la Marne**
**F-33700 Mérignac(FR)**

(72) Inventeur: **Perovitch, Philippe**
**251, avenue de la Marne**
**F-33700 Mérignac(FR)**

(74) Mandataire: **Wagret, Jean-Michel et al**
**CABINET WAGRET 23, rue de Léningrad**
**F-75008 Paris(FR)**

Rank Xerox (UK) Business Services

## Description

La présente invention concerne une composition permettant par application selon une zône déterminée et à l'état liquide ou semi-liquide sur un support notamment sur un tissu corporel vivant, d'assurer la protection de cette zône après sa solidification.

L'invention telle que décrite dans la revendication 1 concerne également un dispositif applicateur permettant la mise en place (de façon utile et pratique par un simple particulier) de la composition destinée à constituer le film de protection.

L'invention trouvera de nombreuses applications chaque fois que l'on veut assurer selon une zône particulière l'isolement d'un tissu, et plus particulièrement du tissu épidermique en contact avec le milieu extérieur afin d'en assurer la protection.

L'invention permet également d'assurer la solidarisation d'un film sur une partie du corps en constituant ainsi un moyen de fixation local ou de mise en place ponctuelle d'un élément actif.

L'invention permet ainsi d'assurer la mise en place et le maintien en position et à l'endroit voulu d'un produit actif déterminé susceptible d'avoir une action sur le substrat constitué du tissu vivant.

L'invention trouvera notamment une application par exemple dans le domaine cosmétologique en permettant de constituer sur les parties de l'épiderme devant subir un traitement spécifique, un film faisant corps avec le tissu support, film dont le degré de souplesse pourra être ajusté en fonction des besoins et le film ainsi réalisé permet d'assurer le maintien en place, pendant une période de temps voulu d'un produit actif.

Le produit actif peut être étranger ou extérieur au film ainsi mis en place ; ce pourrait être le cas par exemple d'un réactif (du type destiné à permettre de caractériser une réaction biochimique ou biologique) ; le produit peut également être présenté sous forme de poudre, ou de pastilles devant rester au contact du tissu pendant une durée déterminée et qui sont ainsi maintenues en place étant emprisonnées dans la capsule que constitue le film, adhérant au tissu support et enfermant le produit actif maintenu au contact de la peau.

Mais le produit actif peut également être incorporé dans la masse même de la composition plastique et être ainsi exsudé à partir du film après solidification et ainsi libéré lentement pour être amené au contact de l'épiderme ou du tissu sur lequel se trouve placé le film après solidification.

On connaît dans la pharmacopée traditionnelle l'utilisation de compositions plastiques formées notamment de collodion (nitrocellulose dans un solvant constitué de mélange alcool/éther) le collodion étant appliqué notamment sur des parties agressées de l'organisme pour en assurer la protection et la fermeture.

Cependant cette technique n'a pas pu se développer et se généraliser malgré l'intérêt qu'elle pouvait présenter sur le plan pratique étant donné les difficultés de mise en oeuvre.

Il est en effet nécessaire que la composition à base de collodion puisse être délivrée et appliquée de façon sensiblement uniforme sur la partie du tissu qu'elle soit recouvrir et auquel elle doit adhérer.

Or, il n'est pas commode, voir même impossible pour l'utilisateur particulier, surtout s'il se trouve précisément handicapé par l'agression physique subie et qu'il doit soigner, d'appliquer une couche uniforme de collodion pour réaliser le film ou la pellicule protectrice.

Ceci d'autant plus que le plus fréquemment la plaie ou l'agression physique à soigner se situe au niveau d'une main ; dans ces conditions la mise en place d'une solution à l'état liquide de façon à réaliser un film uniforme, par exemple à partir d'une brosse ou d'un pinceau préalablement trempé dans la réserve de liquide, ne représente pas une opération commode et réconfortante pour le sujet.

La présente invention permet précisément de réaliser la mise en place d'un film de protection sur une zône de la peau à protéger notamment dans le cas d'une plaie ou de tissu ayant subi une altération et un traumatisme local.

Mais l'invention reçoit (comme exposé ci-dessus) de nombreuses applications chaque fois que l'on voudra assurer la protection d'une zône de l'épiderme tout en évitant une gêne ou un handicap dans l'utilisation du membre ou de l'organe correspondant.

A cet effet l'invention concerne une composition plastique apte à être appliquée sur un substrat épidermique dont elle épouse la configuration, auquel elle est solidarisée en couche mince par sa solidification rapide et spontanée et dont elle assure la protection étanche, la composition étant caractérisée en ce qu'elle est constituée de collodion conditionné en tube compressible comportant un dispositif d'application et ainsi apte à être distribué sous forme de film d'épaisseur uniforme sur la zone réceptrice.

La composition comporte en outre un produit actif apte à agir sur le substrat épidermique, le produit atif étant ainsi maintenu en place et rendu disponible pour agir sur ledit substrat aussi longtemps que le film protecteur est maintenu en situation d'adhérence sur le substrat.

L'invention concerne également un dispositif pour l'application locale de la composition ci-dessus et le dispositif est caractérisé en ce qu'il est constitué d'un tube compressible pourvu d'un orifice pour exprimer la composition à l'état fluide, l'orifice étant associé à une lame souple formant spatule d'application directionnelle apte à guider et contrôle le positionnement du film plastique solidifiable.

Le document FR-A-1 113 147 offre des exemples de dispositifs similaires.

Le dispositif ci-dessus comporte une spatule d'application constituée d'une lame d'épaisseur décroissante vers son bord terminal libre formant une arête souple, l'orifice d'expression de la composition étant disposé à la base, et à proximité de la paroi dans sa zone la plus épaisse de ladite spatule d'application de sorte que la composition soit étalée par déplacement de la spatule dans la direction de l'orifice d'expression.

La spatule comporte sur sa face active d'application et de dispersion, tournée vers l'orifice d'expression de la composition, deux rebords latéraux propres à maintenir sous la spatule la composition en cours d'application en évitant le débordement hors de la zone à couvrir et définie par la largeur de la spatule.

Selon une forme particulière de réalisation la composition ci-dessus comporte un produit actif à action biologique notamment apte à assurer la régénération des tissus vivants sur lesquels le film est appliqué.

Selon une autre forme de réalisation la composition à base de collodion comporte un agent plastifiant apte à ajuster et moduler la souplesse du film solidifié, cet agent étant choisi dans la famille des corps gras à l'état liquide.

L'invention concerne également un dispositif tel que précisé ci-dessus et dans lequel le tube, associé à la spatule ou languette d'application comporte un capuchon de fermeture apte à assurer le recouvrement de ladite spatule d'application et le capuchon comporte également un tenon apte à pénétrer et à obturer l'orifice de sortie de la composition à base de collodion.

Selon une autre caractéristique de l'invention, le capuchon comporte un dispositif de verrouillage de sécurité apte à assurer le maintien du capuchon en position de fermeture, et ce dispositif est constitué d'un élément rétractable formant penne apte à être engagé à force dans une gâche, de sorte que le déverrouillage du capuchon en vue de dégager l'orifice et la spatule d'application, nécessite une pression exercée sur ledit élément rétractable de façon à provoquer sa rétraction et à le dégager par rapport à ladite gâche.

D'autres caractéristiques et avantages de l'invention ressortiront de la description qui suit et qui est donnée en rapport avec une forme de réalisation particulière et en se référant aux dessins annexés.

La figure 1 représente une vue en perspective du dispositif d'application selon la présente invention en position de fermeture.

La figure 2 représente une vue du dispositif applicateur selon l'invention montrant la face active de la spatule de répartition et d'égalisation du film.

La figure 3 représente une vue identique à la figure 2 mais présentant le dispositif applicateur selon la face opposée à la face représentée sur la figure précédente.

Selon l'ensemble des figures le système d'application conformément à l'invention comporte une réserve formée d'un tube compressible de façon connue en soi ; ce tube 1 contient la réserve de la composition selon l'invention c'est-à-dire du collodion incorporant éventuellement un produit actif apte à exercer une action sur l'épiderme ou sur tout substrat sur lequel le film de collodion sera appliqué.

Le réservoir 1 est donc formé d'une paroi souple en forme de sac 2 associé à une tête 3 disposée transversalement par rapport à l'axe longitudinal de la poche ou sac 2 formant le réservoir 1.

La tête 3 est elle-même solidaire d'une spatule d'application 4 formée d'une languette ou lame souple de profil général allant en s'amenuisant depuis sa partie proximale 4a raccordée à la tête 3 jusqu'à la partie distale 4b qui forme l'arête ou bord terminal d'une épaisseur réduite à quelques dixièmes de millimètres.

Dans ces conditions la spatule d'application se prête au mouvement grâce à sa grande souplesse propre.

Par exemple l'ensemble du réservoir et de la tête ainsi que la spatule 4 peuvent être réalisés en polyéthylène basse pression ; les différentes parties sont alors moulées d'une seule pièce et le remplissage se fait par l'extrémité terminale 2a du sac 2 qui est obturé par repli ou soudure transversale de façon connue en soi.

La tête 3 comporte dans sa partie sensiblement centrale et à proximité de la face 4c, qui constitue la face active de la spatule 4 un orifice 5 pour la sortie de la solution ou composition contenue à l'intérieur du sac 2 formant réservoir.

Par pression sur les parois du sac 2 la solution fluide contenue dans ce réservoir est donc exprimée par le conduit intérieur aboutissant à l'orifice 5.

Le conduit situé à l'intérieur et longitudinalement dans la tête 3 et se prolongeant par la protubérance 6 solidaire de la tête 3 jusqu'à l'orifice 5 a une section décroissante depuis son entrée débouchant à l'intérieur du réservoir, jusqu'à l'orifice de sortie 5.

De sorte que la quantité de solution de la composition fluide qui est exprimée par pression sur les parois du sac réservoir 2 restent limitée et peut être ajustée de façon à permettre le dosage régulier de l'arrivée du fluide soumis à étalement par la spatule 4, dont la face active 4c est au contact de la zône à recouvrir.

Sur le corps du réservoir vient s'emboîter le capuchon 7 formé d'un corps de recouvrement apte à contenir la languette 4 et à venir s'appliquer contre la paroi transversale de la tête 3.

Un système d'obturation connu en soi et constitué de la tige 8 pénètre dans l'orifice 5 de façon à éviter la formation d'une "carotte" par solidification d'un résidu de solution à l'intérieur du conduit aboutissant à l'orifice 5.

La tige d'obturation 8 est elle-même venue d'une paroi transversale 9 disposée dans le fond d'une niche 10 venant s'appliquer sur la protubérance 6 de façon à assurer l'obturation complète de l'ensemble.

Le dispositif comporte en outre un verrouillage de sécurité formé des deux dents jumelles 11 et 11′ dont l'extrémité comporte une saillie vers l'extérieur respectivement 12 et 12′ ; les deux dents jumelées 11 et 11′ sont susceptibles de se rétracter l'une vers l'autre pour permettre l'engagement des deux dents jumelées et formant pennes dans la gâche 13.

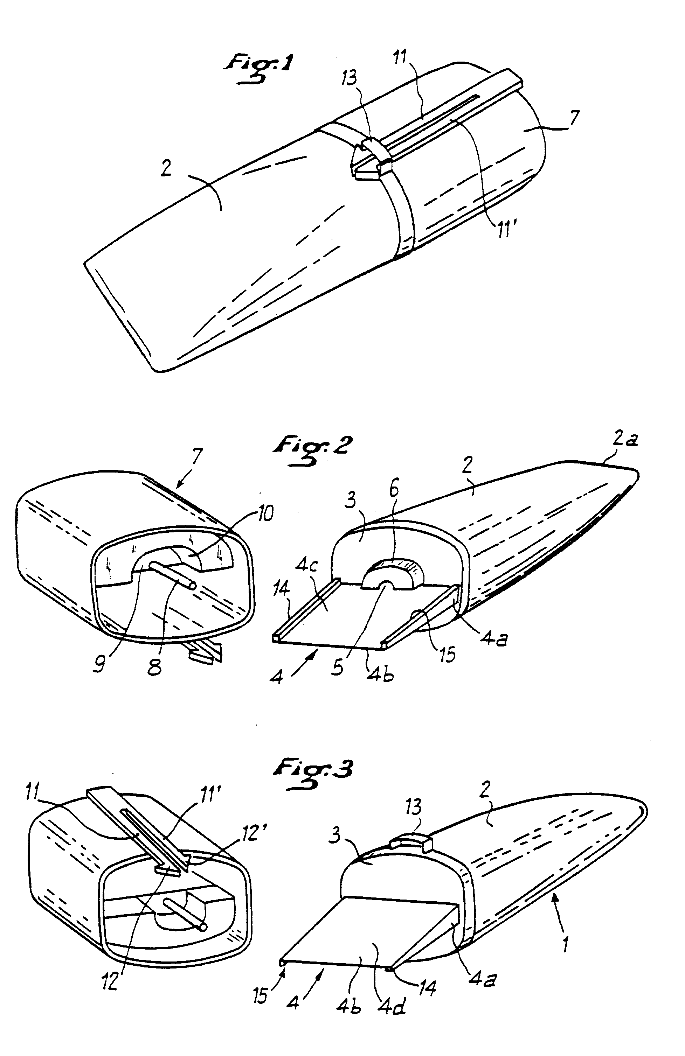

On voit que la fermeture se fait ainsi par engagement des dents jumelées 11 et 11′ dans la gâche 12 ; la poussée longitudinale provoque le rapprochement des dents 11 et 11′ et leur engagement en position d'encliquetage dans la gâche 13, l'écartement par rappel élastique des dents 11 et 11′ provoquant le verrouillage de l'ensemble comme représenté à la figure 1 ; de sorte que la poussée de rétraction exercée sur le capuchon est contrecarrée par le verrouillage et ne permet pas une ouverture accidentelle.

Ce dispositif permet de conserver sans précaution exceptionnelle l'ensemble du dispositif applicateur dans une trousse, sac à main, pharmacie de secours, bagage, vide-poche etc... ; on ne risque pas ainsi une ouverture accidentelle avec perte de la solution fluide venant se solidifier accidentellement par évaporation du solvant (éther et alcool).

On évite également dans ces conditions l'utilisation et l'inhalation de vapeurs nocives par les jeunes enfants.

On a compris que la spatule d'application 4 comporte une face inactive 4d représentée notamment exposée à la figure 3 et qui est au recto de la face active 4c.

Sur la face active 4c vient s'appliquer la solution fluide exprimée par l'orifice 5 et en cours d'étalement sur le substrat.

Selon l'invention les bords latéraux de cette face active 4c comportent deux retours 14,15 (fortement agrandis sur la figure 2 pour la clarté du dessin).

Ces rebords permettent de maintenir et de canaliser la phase fluide exprimée par l'orifice 5 sur la paroi de la surface d'application constituée par la face 4c de la spatule 4, la couche de fluide étant emprisonnée entre le substrat récepteur, la face active 4c de la spatule d'application et latéralement par les deux rebords 14 et 15.

Dans ces conditions le dispositif permet d'obtenir la répartition et la mise en place d'un film d'épaisseur uniforme sur le substrat ; cette épaisseur est en effet ajustée par le jeu de la languette 4, grâce aux bords 14,15 latéraux qui permettent de maintenir l'arête terminale 4 à une distance exactement réglée par rapport au substrat en laissant ainsi se répandre, après rétraction de la languette ou spatule 4 vers l'arrière, le film d'épaisseur convenable.

Et de plus les rebords 14,15 évitent que la composition fluide ne puisse se répandre et s'étaler en dehors de la zône couverte par la spatule.

On comprend que dans ces conditions le dispositif et la composition selon l'invention contenue dans ce dispositif permettent l'utilisation de collodion à des fins multiples notamment dans le cadre de la mise en place d'une pellicule de protection souple sur un tissu épidermique.

L'invention permet de façon nouvelle de mettre ainsi à la disposition des utilisateurs une composition et un dispositif d'application qui lui est associé pour permettre la mise en place d'une pellicule de protection notamment sur la peau en tout endroit du corps humain sans gêne particulière.

Dans le cas où le produit aura été associé, conformément à l'invention, à un produit actif notamment régénérateur des tissus et apte à apporter pendant une durée convenablement dosée une action de régénération locale, on pourra ainsi réaliser à des fins dermatologiques ou cosmétologiques une application pour remédier à des insuffisances localisées ou à une fatigue ponctuelle du tissu.

Le film solide obtenu conformément au dispositif et aux caractéristiques de l'invention reste en effet sur place aussi longtemps qu'il est maintenu sans être retiré et il permet donc de stocker et d'apporter de façon continue au substrat les éléments par exemple nutritifs ou susceptibles de provoquer une reconstitution tissulaire ; en même temps la pellicule ou film solide maintenu en place peut permettre outre une protection physique du substrat, une remise en forme ; par exemple dans le cas d'une mise en place d'un film comportant une structure suffisamment rigide (éventuellement par application de plusieurs films successifs) on peut obtenir un effet physique de correction susceptible d'exercer par exemple une action anti-rides en contrecarrant la formation d'un pli dans l'épiderme.

Dans le cas d'une agression provoquant une interruption accidentelle dans l'intégrité et la continuité du tissu, l'application du film de protection selon l'invention permet d'obtenir un effet cumulé de protection contre les souillures et l'infection par les germes provenant de l'extérieur, un effet de protection physique contre les agressions liquides, lorsque le membre (notamment la main) ainsi protégé doit affronter, par suite de travaux professionnels ou ménagers un milieu susceptible de contrecarrer la cicatrisation ; ce peut être le cas de liquides mais également de produits à l'état pulvérulent.

A cet égard le film de protection solide appliqué conformément à l'invention permet de remplacer les pansements sous forme de bandelettes ou sous forme de tissu caoutchouté auto-adhésif utilisé de façon habituelle.

Et l'invention aura donc une application particulière et fréquente dans le cas des petites plaies linéaires des mains et des doigts qui sont les plus fréquentes.

Plus spécialement l'utilisation du film de protection selon l'invention sera notamment avantageuse dans le cas des professionnels travaillant dans les industries de précision ou lesquelles un pansement classique constitue une gêne.

L'invention sera également utilisée de façon avantageuse dans le cas de professionnels astreints (notamment dans les indutries alimentaires, biologiques ou pharmaceutiques) à une rigoureuse propreté des mains ; dans ce cas la position d'un pansement recouvrant la plaie outre qu'il représente une gêne pour l'exécution des travaux, n'est pas compatible avec les risques de contamination ; il serait alors nécessaire pour le professionnel d'utiliser un gant en caoutchouc venant recouvrir l'ensemble de la main mais qui présente alors l'inconvénient de faire macérer la plaie ou la blessure.

On citera également les corps de métiers manipulant des matériaux salissants ou agressifs pour la plaie, notamment dans le cadre des industries du bâtiment manipulant des produits pulvérulents tels que ciment, platre, chaux, ou encore les agriculteurs (engrais).

On mentionnera également l'utilisation avantageuse de l'applicateur et de la solution selon l'invention dans le cadre des profesions médicales ou paramédicales pour lequel le port d'un pansement est rendu indispensable par les risques de contamination extérieure en milieu hospitalier, alors que l'application d'un pansement traditionnel n'assurerait qu'une protection incomplète à moins de prévoir des conditions sévères entraînant une gêne dans la dextérité manuelle.

L'invention sera surtout et particulièrement utile pour le particulier apte à subir des lésions superficielles mineures dans la vie courante mais pour lesquelles une plaie même de faible importance contitue une gêne.

L'application du film fluide selon l'invention permet pratiquement au sujet d'oublier la plaie celle-ci étant totalement protégée tout en permettant la respiration du tissu et le libre mouvement du corps.

Les actes de la vie courante peuvent être effectués et l'exercice manuel peut être pratiqué de façon habituelle sans nécessiter de contraintes particulières (notamment en présence de liquide).

Ces conditions doivent être ressenties avantageusement par le sujet notamment en période de vacances au bord de la mer, immersion de la plaie à l'occasion de la baignade dans le milieu salin étant douloureuse et contrecarrant la cicatrisation.

Le film protecteur selon l'invention étant en outre transparent peut donc être utilisé par tous les sujets quelle que soit la couleur de leur peau, sans entraîner de contraste disgracieux.

Le film protecteur selon l'invention sera également utilisé auprès des sujets récalcitrants par exemple les enfants ou les animaux sans risque de réaction instinctive de rejet, et sans nécessiter une surveillance attentive de la situation du pansement.

Le film de protection appliqué conformément à l'invention pourra être conservé aussi longtemps que nécessaire puisqu'il permet à la plaie de respirer et de cicatriser rapidement.

Le film appliqué conformément à l'invention permet, outre l'effet de protection physique, d'assurer le maintien en place des tissus et peut donc avantageusement remplacer le mise en place des points de suture traditionnels, pour des petites plaies rectilignes ne dépassant pas quelques centimètres de longueur (3-4 cm), car les points de suture sont fréquemment douloureux et susceptibles de laisser des traces disgracieuses.

Au contraire le film de protection selon l'invention permet d'obtenir le rapprochement des bords de la plaie et leur solidarisation selon une ligne continue et évitant ainsi l'apparition de séquelles dans l'esthétique de l'épiderme.

La composition selon l'invention pourra avantageusement comporter dans sa masse des produits actifs susceptibles par une activation dans la construction tissulaire de provoquer et faciliter la fermeture de la lésion.

Le collodion étant constitué d'une solution de nitrocellulose dans un mélange éther-alcool, suppose dès son application un effet de désinfection par l'action superficielle de la phase éther-alcool avant sa volatilisation.

Le film protecteur peut être renouvelé autant de fois que nécessaire soit après enlèvement du film ancien et application d'une nouvelle couche de la composition engendrant un nouveau film de protection, soit par mise en place d'une couche superficielle additionnelle débordant au-delà du premier film et assurant ainsi un renforcement et un maintien en place du film initial.

La présente invention est donc applicable de façon préférentielle à la réalisation d'un pansement de protection dans des applications multiples allant des interventions pratiquées ou effectuées en milieu médical aux soins effectués à domicile par l'utilisateur particulier.

Dans le cadre de cette application pour la mise en place d'un pansement le film protecteur obtenu conformément à l'invention présente de nombreux avantages par rapport aux pansements traditionnels.

On notera en premier lieu que le pansement selon l'invention étant transparent permet le passage de flux lumineux qui sont un facteur de cicatrisation ; alors que cependant le pansement formé du film de protection selon l'invention étant perméable à l'air permet également à la plaie de respirer.

Ce caractère transparent est également avantageux en ce qu'il permet aux intéressés (le patient ou le praticien) de surveiller l'évolution de la plaie sans avoir à défaire puis refaire le pansement.

Le pansement selon l'invention évite toute rupture dans la continuité de la surface corporelle et il épouse par conséquent la conformation et le relief naturel du corps ce qui présente un intérêt et un avantage esthétique susceptible d'être apprécié par de nombreux usagers notamment dans le cas où le pansement est appliqué sur des parties normalement visibles du corps.

L'invention permet également de réaliser un pansement susceptible d'être associé à un élément de rigidification sous forme d'un support solide formé d'une structure réticulée ou filamentaire telle qu'une gaze ou une nappe de coton qui pourra être appliquée sur la plaie directement ou après une première couche de mise en place de la composition selon l'invention et la structure solide (coton, gaze) ainsi appliquée sur le premier film protecteur pourra à son tour recevoir une couche de collodion imprégnant cette structure et faisant corps avec la couche inférieure l'ensemble constituant une structure homogène particulièrement solide et susceptible par conséquent d'assurer de façon ferme le maintien en place des tissus notamment la fermeture de la plaie.

Dans tous les cas le pansement réalisé dans le cadre de la présente invention assure un recouvrement uniforme de la zone sur lequel il est appliqué en assurant une solidarisation du film sur toute cette surface ; ceci forme contraste avec les pansements traditionnels notamment les pansements préparés et formés d'une zône centrale venant se superposer sur la plaie associée à deux ailes diamètrement opposées permettant la solidarisation sur les bords périphériques du tissu ; dans ce cas la zône centrale n'adhère pas au tissu support de sorte que cette zone centrale est susceptible de se déplacer indépendamment du substrat tissulaire sur lequel elle est appliquée et qui est constitué précisément par la partie agressée ; on risque ainsi dans le cas de l'application de pansements traditionnels d'aboutir à un frottement régulier, en fonction des mouvements du sujet, du pansement se superposant à la zone blessée ; dans le cas du pansement réalisé conformément à l'invention, le film superficiel adhère sur toute la surface de la partie du tissu vivant agressé en évitant ainsi une zône médiane recouvrant la plaie et qui risquerait d'être déplacé indépendamment du support et de provoquer un frottement particulièrement néfaste à la cicatrisation d'une part et douloureux pour le sujet d'autre part.

On notera enfin que le pansement selon l'invention permet de s'adapter à toute sorte de conformation de plaies puisqu'il est délivré à l'état liquide et qu'il se solidifie sur place ; il peut donc épouser des formes les plus diverses en formant un film continu ; dans le cas par exemple d'une plaie angulaire (en V, en L, en Z, S etc...), il était nécessaire dans le cadre des dispositifs antérieurs soit de réaliser un pansement très large couvrant l'ensemble de la surface, soit de disposer des pansements préparés et formés de bandes linéaires auto-adhésives, dans les différentes directions linéaires correspondant ; et dans le cas d'une plaie de forme courbe ou angulaire il était donc nécessaire d'apposer deux ou trois, voire plus bandes de pansement auto-adhésives ; alors que dans le cadre de la mise en oeuvre de la présente invention le pansement peut être appliqué en épousant de façon parfaitement fidèle toutes les formes si éloignées soient-elles d'une forme linéaire.

On donne ci-après un exemple de préparation de la composition conforme à l'invention.

On prépare une charge de cellulose nitrée (nitrate de cellulose ou fulmicoton) à partir de fibres de coton traitées par un mélange d'acide sulfurique et d'acide nitrique. La cellulose nitrée est mise en solution dans un solvant constitué de méthanol et d'éther.

On utilise entre 30 et 100 grammes de cellulose nitrée pour une solution d'un litre de solvant formé de trois à quatre parts d'éther pour une part de méthanol.

On ajoute facultativement un corps gras sous forme par exemple d'huile de castor dans la proportion de 2 à 3 % du mélange de la solution finale dans le cas où l'on veut obtenir un film flexible.

La solution finale peut être complétée par incorporation de substances à action antiseptique ou de produits susceptibles de procurer une action de régénération des tissus et d'adjuvants à la cicatrisation.

Dans ces produits on citera les substances ci-après définies.

Parmi les produits antiseptiques :

- Mercurothiolate de sodium, entre 0,5 et 1 % et de préférence 1 %
- Eosine, entre 0,5 et 2 % et de préférence 1 %
- Chlorexidine, entre 0,2 et 1 % et de préférence 0,5 %
- Borate de Phénylmercure entre 0,2 et 1 % et de préférence 0,5 %.

Parmi les produits à pouvoir cicatrisant :

- Rétinol, entre 1 et 3 % et de préférence 2,5 %
- Vitamine A, entre 1 et 2 % et de préférence 1,5 %
- Allantoïne, entre 1 et 2 % et de préférence 1,5 %
- Vitamine E (Tocophérol), entre 1 et 2 % et de préférence 1,5 %
- N Acétyl Hydroxyproline, entre 1 et 2 % et de préférence 1,5 %
- Huile essentielle de thym, entre 1 et 3 % et de préférence 3 %
- Huile essentielle de Niaouli, entre 1 et 3 % et de préférence 2 %
- Extrait de Centella Asiatica, entre 2 et 4 % et de préférence 3 %
- Papaïne, entre 0,2 et 1 % et de préférence 0,5 %
- Dérivés benzoïques et Cinnamiques du Baumier du Pérou entre 1 et 2 % et de préférence 1,5 %
- Tanins galliques d'Hamamélis, entre 1 et 2 % et de préférence 1,5 %
- Essence de Romarin (Monoterpènes Bicycliques Alpha-Pinène, Bornéol), entre 0,5 et 2 % et de préférence 1 %
- Essence de Sauge (Monoterpènes bicycliques : Thuyone), entre 0,5 et 2 % et de préférence 1 %.

De nombreuses combinaisons associatives sont possibles parmi ces divers produits afin d'obtenir l'effet souhaité.

Dans le cas de l'application de la composition de l'invention à la mise en place d'un pansement sur une plaie, impliquant par conséquent la formation d'un caillot à partir du sang coagulé au niveau des bords de la plaie, la mise en place du pansement ainsi réalisée permet d'éviter le phénomène de durcissement et de rétraction sur lui-même du caillot au fur et à mesure du séchage de ce dernier comme cela se passe dans le cadre d'un pansement textile ; ce phénomène de rétraction du caillot présente l'inconvénient de durcir la plaie et de former une croûte qui fréquemment aboutit à gêner l'évolution de la plaie vers sa cicatrisation par formation et reconstitution du tissu cutané ; dans le cadre du pansement réalisé conformément à l'invention le caillot se forme et joue son rôle d'obturation et de protection physique de la plaie sans cependant aboutir, par suite d'une dessication trop rapide à la constitution d'un "corps étranger" gênant la fermeture et la reconstitution tissulaire sous-jacente.

**Revendications**

1. Ensemble pour la mise en place d'un film protecteur notamment sur un substrat épidermique comprenant en combinaison les points suivants:

   a) il est constitué d'une réserve compressible (1) pourvue d'un orifice (5) pour exprimer une composition à l'état fluide contenue dans la réserve, cette réserve étant constituée par un tube (2)

   b) cet orifice (5) est associé à une spatule d'application (4) directionnelle apte à guider et contrôler le positionnement et l'épaisseur d'un film formé de la dite composition,

   cet ensemble étant caractérisé en ce que:

   - la réserve contient une composition à base de collodion, le film de collodion déposé par la spatule étant solidifiable sur son substrat épidermique auquel il est solidarisé en couche mince et dont il assure la protection,
   - la composition comporte en outre un produit actif apte à agir sur le substrat épidermique, le produit actif étant ainsi maintenu en place et rendu disponible pour agir sur ledit substrat aussi longtemps que le film protecteur est maintenu en situation d'adhérence sur le substrat,
   - la spatule d'application (4) est constituée d'une lame d'épaisseur décroissante vers son bord terminal libre formant une arête souple (4b), l'orifice d'expression (5) de la composition étant disposé à la base et à proximité de la paroi dans sa zone la plus épaisse de ladite spatule d'application (4) de sorte que la composition soit étalée par déplacement de la spatule dans la direction de l'orifice d'expression, deux rebords latéraux parallèles (14) et (15) propres à maintenir sous la spatule la composition en cours d'application en évitant le débordement hors de la zone à couvrir et définie par la largeur de la spatule,

- l'ensemble comporte un capuchon (7) de fermeture apte à assurer le recouvrement de ladite spatule d'application (4) et le capuchon comporte également un système d'obturation formé par un tenon (8) apte à pénétrer et à obturer l'orifice de sortie (5) de la composition à base de collodion et une niche (10) fermée par une paroi (9), cette niche (10) étant destinée à s'appliquer sur une protubérance (6) de forme complémentaire de la face (3) du tube (2).

2. Ensemble selon la revendication 1, caractérisé en ce que la composition comporte un produit actif à action biologique, notamment antiseptique, de régénération des tissus et/ou de cicatrisation.

3. Ensemble selon l'une des revendications 1 ou 2 caractérisé en ce qu'il comporte un agent plastifiant apte à ajuster et moduler la souplesse du film solidifié, cet agent étant choisi dans la famille des corps gras à l'état liquide, notamment de l'huile de ricin.

4. Ensemble selon la revendication 1, caractérisé en ce que le capuchon (7) comporte un dispositif de verrouillage de sécurité apte à assurer le maintien du capuchon en position de fermeture, et ce dispositif est constitué d'un élément rétractable formant penne (11, 11') apte à être engagé à force dans une gâche (13), de sorte que le déverrouillage du capuchon, en vue de dégager l'orifice (5) et la spatule d'application (4), nécessite une pression exercée sur ledit élément rétractable de façon à provoquer sa rétraction et à le dégager par rapport à ladite gâche.

5. Application d'un ensemble, selon l'une des revendications 1 à 4 ci-dessus, pour la réalisation d'un pansement de protection auto-adhérant à l'épiderme et épousant exactement la conformation de la plaie et de la zone à protéger.

6. Application selon la revendication 5 ci-dessus pour conformer un pansement, caractérisé en outre en ce que le film ainsi mis en place est transparent permettant l'action conjuguée de l'air et de la lumière pour favoriser la cicatrisation.

**Claims**

1. Assembly for positioning a protective film in particular on an epidermic substrate comprising, in combination the following points:
   a) it is constituted by a compressible reserve 1 provided with an orifice 5 for expressing a composition in the fluid state contained in the reserve, this reserve being constituted by a tube 2;
   b) this orifice 5 is associated with a spatula 4 for directional application adapted to guide and control the positioning and thickness of a film formed from said composition,
   this assembly being characterized in that:
   - the reserve contains a composition based on collodion, the film of collodion deposited by the spatula being adapted to solidify on its epidermic substrate to which it adheres in a thin layer and of which it ensures protection,
   - the composition further comprises an active product adapted to act on the epidermic substrate, the active product thus being maintained in place and rendered available to act on said substrate for as long as the protective film is maintained in situation of adherence on the substrate,
   - the spatula 4 for application is constituted by a blade whose thickness decreases towards its free terminal edge forming a supple edge 4b, the orifice 5 for expressing the composition being disposed at the base and in the vicinity of the wall in the thickest zone of said spatula 4 for application, with the result that the composition is spread by displacement of the spatula in the direction of the expression orifice, two parallel lateral flanges 14 and 15 adapted to maintain beneath the spatula the composition in the course of application, avoiding overflow out of the zone to be covered and defined by the width of the spatula,
   - the assembly comprises a cap 7 for closure, adapted to cover said spatula 4 for application, and the cap also comprises an obturation system formed by a peg 8 adapted to penetrate in and to obturate the orifice 5 for expression of the composition based on collodion and a recess 10 closed by a wall 9, this recess 10 being adapted to be applied on a protuberance 6 of complementary shape on the face 3 of the tube 2.

2. Assembly according to Claim 1, characterized in that the composition comprises an active product with biological action, particularly antiseptic, for regeneration of the tissues and/or for cicatrization.

3. Assembly according to one of Claims 1 or 2, characterized in that it comprises a plasticizing agent adapted to adjust and modulate the suppleness of the solidified film, this agent being selected from the family of fats in the liquid state, particularly castor oil.

4. Assembly according to Claim 1, characterized in that the cap 7 comprises a safety locking device adapted to ensure that the cap is maintained in closed position, and this device is constituted by a retractable element forming bolt 11, 11' adapted to be engaged by force in a clasp 13, with the result that unlocking of the cap, with a view to revealing the orifice 5 and the spatula 4 for application, necessitates a pressure exerted on said retractable element so as to provoke retraction thereof and to disengage it with respect to said clasp.

5. Application of an assembly according to one of Claims 1 to 4 hereinabove, for making a protective dressing which is self-sticking on the epidermis and exactly follows the shape of the wound and the zone to be protected.

6. Application according to Claim 5 hereinabove to form a dressing, characterized in addition in that the film thus placed in position is transparent, allowing the combined action of air and light in order to promote cicatrization.

**Patentansprüche**

1. Vorrichtung für die Auftragung eines Schutzfilms, insbesondere auf ein epidermales Substrat, die die Kombination folgender Merkmale umfaßt:

a) sie besteht aus einem komprimierbaren Behälter (1), versehen mit einer Öffnung (5), um eine in dem Behälter sich befindende flüssige Substanz auszupressen, wobei der Behälter aus einer Tube (2) besteht,

b) die Öffnung (5) ist verbunden mit einem Spatel (4) zum Auftragen der Substanz, der dazu geeignet ist, die Richtung und Dicke des Films, der aus der genannten Substanz besteht, zu steuern und zu kontrollieren, dadurch gekennzeichnet, daß:

- der Behälter eine Substanz auf Basis eines Kollodiums enthält, wobei der Kollodiumfilm mit dem Spatel aufgetragen wird und sich auf dem epidermalen Substrat verfestigt, auf das sie mit einer dünnen Schicht aufgebracht wird, und dessen Schutz sie bewirkt,

- die Substanz ein Aktivprodukt enthält, das sich dazu eignet, auf das epidermale Substrat einzuwirken, wobei das Aktivprodukt an der Stelle gehalten und wirksam ist, und auf das genannte Substrat so lang einwirkt, wie der auf das Substrat aufgetragene Schutzfilm haftet,

- der Applikations-Spatel (4) aus einer Klinge mit in Richtung des freien, eine weiche Kante (4b) bildenden Endstücks, abnehmender Dicke, besteht, wobei die Ausgangsöffnung (5) für die Substanz an der Basis und in der Nähe der Wand in der dicksten Zone des Applikations-Spatels (4) derart angebracht ist, daß die Substanz durch eine Verschiebung des Spatels in Richtung der Auspreß-Öffnung verteilt wird, und die beiden parallelen Seitenkanten (14) und (15) geeignet sind, die Substanz während der Applikation unter dem Spatel zu halten, wodurch ein Überlaufen aus der zu bedeckenden Zone, die durch die Größe des Spatels definiert wird, verhindert wird,

- eine Verschlußkappe (7) vorgesehen ist, die sich zum Abdecken des vorgenannten Applikations-Spatels (4) eignet, und daß die Kappe außerdem ein Verschlußsystem enthält, welches aus einem Zapfen (8) gebildet ist, der sich zum Einführen in die und Verschließen der Austrittsöffnung (5) für die Substanz auf Kollodium-Basis eignet, und eine Ausnehmung (10) aufweist, die durch eine Wand (9) abgeschlossen ist, wobei diese Ausnehmung (10) dazu vorgesehen ist, einen entsprechenden Vorsprung (6) aufzunehmen, der an der Vorderseite (3) der Tube (2) angeformt ist.

2. Vorrichtung nach Anspruch 1, dadurch gekennzeichnet, daß die Substanz ein Aktivprodukt mit biologischer, insbesondere antiseptischer Wirkung enthält zur Regenerierung von Geweben und/oder Vernarbungen.

3. Vorrichtung nach Anspruch 1 oder 2, dadurch gekennzeichnet, daß sie einen plastifizierenden Wirkstoff enthält, der sich zum Anpassen und Modulieren der Geschmeidigkeit des verfestigten Films eignet, wobei dieser Wirkstoff aus der Familie der flüssigen Fette ausgewählt ist, insbesondere Rizinusöl.

4. Vorrichtung nach Anspruch 1,

dadurch gekennzeichnet, daß die Verschlußkappe (7) eine Sicherheits-Verriegelungsvorrichtung enthält, die die Kappe bei Nichtgebrauch verschlossen hält, wobei diese Vorrichtung ein rückziehbares Element in Form einer Feder (11, 11') enthält, die durch Krafteinwirkung in ein Schließstück (13) eingefügt werden kann, so daß für die Entriegelung der Verschlußkappe, womit die Öffnung und der Applikations-Spatel freigemacht werden, ein Druck auf dieses rückziehbare Element ausgeübt werden muß, um seine Rückstellung zu bewirken, und um es von dem genannten Schließstück freizumachen.

5. Verwendung einer Vorrichtung nach einem der Ansprüche 1 bis 4, für die Herstellung eines auf der Haut selbsthaftenden Schutzverbandes, der sich dazu eignet, sich genau an die Wunde und die zu schützende Zone anzupassen.

6. Verwendung nach Anspruch 5 zur Herstellung eines Verbandes,
dadurch gekennzeichnet, daß der so aufgebrachte Film transparent ist, um die Einwirkung von Luft und Licht zur Begünstigung der Narbenheilung zu ermöglichen.

Fig.1

Fig.2

Fig.3